# EUROPÄISCHE PATENTSCHRIFT

(11) **EP 1 064 911 B1**
(45) Veröffentlichungstag und Bekanntmachung des Hinweises auf die Patenterteilung: **04.02.2004**
(21) Anmeldenummer: 99112669.9
(22) Anmeldetag: 02.07.1999
(51) Int. Cl.: A61K 7/00, C11D 17/08, A61K 9/50, C08L 5/00

(54) **Mikrokapseln - II**
Microcapsules
Microcapsules

(43) Veröffentlichungstag der Anmeldung: 03.01.2001
(73) Patentinhaber: Cognis Iberia, S.L., 08755 Castellbisbal, (Barcelona) (ES)
(72) Erfinder: Garces, Garces Josep, 08760-Martorell, Barcelona (ES); Viladot Petit, Josep-Lluis, 08036-Barcelona (ES)
(74) Vertreter: Fabry, Bernd, Dr.

(56) Entgegenhaltungen:
- EP-A- 0 152 898
- EP-A- 0 237 542
- WO-A-98/43609
- FR-A- 2 699 545
- US-A- 5 089 272
- US-A- 5 753 264
- US-A- 5 855 904
- PATENT ABSTRACTS OF JAPAN vol. 13, no. 190 (C-593), 8. Mai 1989 (1989-05-08) & JP 01 018440 A (DAINIPPON PHARMACEUT CO LTD), 23. Januar 1989 (1989-01-23) & DATABASE WPI Week 198909 Derwent Publications Ltd., London, GB; AN 65806

## Beschreibung

### Gebiet der Erfindung

Die Erfindung befindet sich auf dem Gebiet der Verkapselung von Wirkstoffen und betrifft neue Mikrokapseln, ein Verfahren zu deren Herstellung unter Einsatz von verschiedenen Polymeren und Chitosanen sowie deren Verwendung zur Herstellung beispielsweise oberflächenaktiver Zubereitungen.

### Stand der Technik

Unter dem Begriff "Mikrokapsel" werden sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden flüssige Wirkstoffe in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammem angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide).

In diesem Zusammenhang sei auch auf die deutsche Patentanmeldung **DE 19712978 A1** (Henkel) hingewiesen, aus der Chitosanmikrosphären bekannt sind, die man erhält, indem man Chitosane oder Chitosanderivate mit Ölkörpern vermischt und diese Mischungen in alkalisch eingestellte Tensidlösungen einbringt. Aus der deutschen Patentanmeldung **DE 19756452 A1** (Henkel) ist ferner auch die Verwendung von Chitosan als Verkapselungsmaterial für Tocopherol bekannt.

Die Freisetzung der Wirkstoffe aus den Mikrokapseln erfolgt üblicherweise während der Anwendung der sie enthaltenden Zubereitungen durch Zerstörung der Hülle infolge mechanischer, thermischer, chemischer oder enzymatischer Einwirkung. Von Nachteil ist dabei, daß die Mikrokapseln die kontrollierte Freisetzung der Wirkstoffe aus ihrem Innem nicht oder nur in unzureichendem Maße zulassen und die Kapseln eine ungenügende Stabilität in Gegenwart von Tensiden, zumal anionischen Tensiden aufweisen. Die Aufgabe der vorliegenden Erfindung hat somit darin bestanden, gerade diese Nachteile zu überwinden.

### Beschreibung der Erfindung

Gegenstand der Erfindung sind Mikrokapseln mit mittleren Durchmessern im Bereich von 0,1 bis 5 mm, bestehend aus einer Hüllmembran und einer mindestens einen Wirkstoff enthaltenden Matrix, die man erhält, indem man
(a) aus Gelbildnem, Chitosanen und Wirkstoffen eine Matrix zubereitet,
(b) die Matrix in einer Ölphase dispergiert,
(c) die dispergierte Matrix mit wäßrigen Lösungen anionischer Polymere behandelt und dabei die Ölphase entfernt.

Überraschenderweise wurde gefunden, daß der Einsatz von thermogelierenden natürlichen Heteropolysacchariden oder Proteinen zusammen mit Chitosanen, die in Gegenwart anionischer Polymere Membranen bilden, die Herstellung von neuen Mikrokapseln erlaubt, die sich durch eine deutlich verbesserte Tensidstabilität auszeichnen.

Ein weiterer Gegenstand der Erfindung betrifft ein Verfahren zur Herstellung von Mikrokapseln mit mittleren Durchmessern im Bereich von 0,1 bis 5 mm, bestehend aus einer Hüllmembran und einer mindestens einen Wirkstoff enthaltenden Matrix, bei dem man
(a) aus Gelbildnern, Chitosanen und Wirkstoffen eine Matrix zubereitet,
(b) die Matrix in einer Ölphase dispergiert,
(c) die dispergierte Matrix mit wäßrigen Lösungen anionischer Polymere behandelt und dabei die Ölphase entfernt.

### Gelbildner

Im Sinne der Erfindung werden als Gelbildner vorzugsweise solche Stoffe in Betracht gezogen, welche die Eigenschaft zeigen in wäßriger Lösung bei Temperaturen oberhalb von 40 °C Gele zu bilden. Typische Beispiele hierfür sind Hetereopolysaccharide und Proteine. Als thermogelierende **Heteropolysaccharide** kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farbals auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wäßriger Lösung Gele bilden, die nicht unterhalb von 80 °C schmelzen und sich bereits oberhalb von 40 °C wieder verfestigen. Aus der Gruppe der thermogelierenden **Proteine** seien exemplarisch die verschiedenen Gelatine-Typen genannt.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

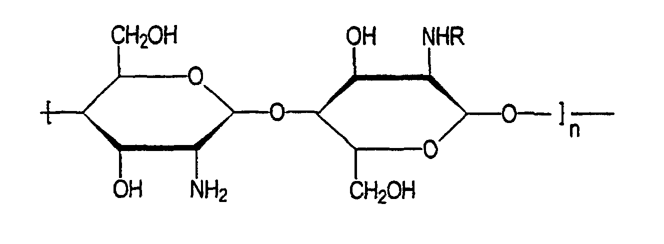

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-232).** Übersichten zu diesem Thema sind auch beispielsweise von B. Gesslein et al. in **HAPPI 27, 57 (1990),** O. Skaugrud in **Drug Cosm.Ind. 148, 24 (1991)** und E. Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR 2701266 A** bekannt. Vorzugsweise werden solche Typen eingesetzt, wie sie in den deutschen Patentanmeldungen **DE 4442987 A1** und **DE 19537001 A1** (Henkel) offenbart werden und die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Wirkstoffe

Die Auswahl der Wirkstoffe, die in den neuen Mikrokapseln eingeschlossen sind, ist an sich unkritisch. Vorzugsweise handelt es sich um Stoffe, die erst durch mechanische Zerstörung der Mikrokapseln freigesetzt werden. In diesen Fällen kommt den Mikrokapseln die Aufgabe zu, den Kontakt zwischen äußerer Umgebung und Wirkstoff und damit eine chemische Reaktion bzw. einen Abbau zu verhindern. Es kann es sein, daß die in der Kapsel eingeschlossenen Stoffe überhaupt nicht freigesetzt werden sollen und ausschließlich dem Zweck dienen, der Zubereitung ein ästhetisches Äußeres zu verleihen; dies trifft beispielsweise vielfach für Farbstoffe zu. Es ist natürlich klar, daß diese Einsatzformen auch nebeneinander bestehen können. Insbesondere ist es möglich, beispielsweise einen Duftstoff für die spätere Freisetzung zusammen mit einem Farbpigment zu verkapseln, welches der Kapsel ein besonderes Aussehen verleiht.

### Wirkstoffe für kosmetische und pharmazeutische Anwendungen

Typische Beispiele für Wirkstoffe, wie sie im Bereich der kosmetischen und pharmazeutischen Zubereitungen eingesetzt werden sind Tenside, kosmetische Öle, Perlglanzwachse, Stabilisatoren, biogene Wirkstoffe, Vitamine, Deodorantien, Antitranspirantien, Antischuppenmittel, UV-Lichtschutzfaktoren, Antioxidantien, Konservierungsmittel, Insektenrepellentien, Selbstbräuner, Typrosininhibitoren (Depigmentierungsmittel), Parfümöle und Farbstoffe.

Als oberflächenaktive Stoffe können anionische, nichtionische, kationische und/oder amphotere bzw. amphotere Tenside verkapselt werden. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofem die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

Als **kosmetische Öle** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen undloder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
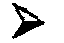 adstringierende Wirkstoffe,
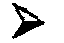 Ölkomponenten,
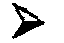 nichtionische Emulgatoren,
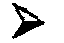 Coemulgatoren,
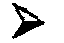 Konsistenzgeber,
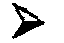 Hilfsstoffe wie z. B. Verdicker oder Komplexierungsmittel und/oder
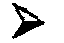 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
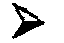 entzündungshemmende, hautschützende oder wohlriechende ätherische Öle,
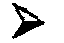 synthetische hautschützende Wirkstoffe und/oder
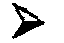 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Climbazol, Octopirox, Ketokonazol und Zinkpyrethion eingesetzt werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
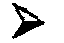 3-Benzylidencampher bzw. 3-Benzylidennorcampher und dessen Derivate, z.B. 3-(4-Methylbenzyliden)campher wie in der **EP 0693471 B1** beschrieben;
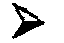 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
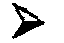 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
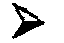 Ester der Salicylsäure, vorzugsweise Salicylsäure-2-ethylhexylester, Salicylsäure-4-isopropylbenzylester, Salicylsäurehomomenthylester;
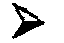 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
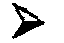 Ester der Benzalmalonsäure, vorzugsweise 4-Methoxybenzmalonsäuredi-2-ethylhexylester;
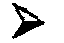 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
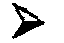 Propan-1,3-dione, wie z.B. 1-(4-tert.Butylphenyl)-3-(4'methoxyphenyl)propan-1,3-dion;
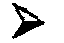 Ketotricyclo(5.2.1.0)decan-Derivate, wie in der **EP 0694521 B1** beschrieben.

Als wasserlösliche Substanzen kommen in Frage:
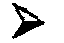 2-Phenylbenzimidazol-5-sulfonsäure und deren Alkali-, Erdalkali-, Ammonium-, Alkylammonium-, Alkanolammonium- und Glucammoniumsalze;
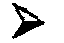 Sulfonsäurederivate von Benzophenonen, vorzugsweise 2-Hydroxy-4-methoxybenzophenon-5-sulfonsäure und ihre Salze;
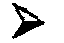 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton. Als **Tyrosinhinbitoren,** die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Unalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Wirkstoffe können auch ausschließlich aus ästhetischen Gründen in den Kapseln enthalten und nicht für eine kontrollierte Freigabe vorgesehen sein.

### Wirkstoffe für Detergensanwendungen

Bei Mikrokapselanwendungen im Bereich der Detergentien, insbesondere bei Wasch- und Reinigungsmitteln besteht ebenfalls der Wunsch, den Kontakt der verschiedenen Einsatzstoffe miteinander zu verhindern. So ist es sinnvoll, chemisch empfindliche Stoffe, wie beispielsweise Parfümöle oder optische Aufheller zu verkapseln, um deren Aktivität beispielsweise in Chlor- oder Peroxidbleichlaugen auch bei längerer Lagerung sicherzustellen. Man nutzt jedoch beispielsweise auch den Effekt, daß die Bleiche von Textilien in der Regel nicht zu Beginn des Waschprozesses, sondern erst in dessen Verlauf stattfindet und stellt mit der durch mechanische Einwirkung auf die Mikrokapseln verzögerten Freisetzung sicher, daß die Bleichmittel zum richtigen Zeitpunkt ihre volle Wirkung entfalten. Demzufolge kommen als Wirkstoffe, die es für Detergensanwendungen zu verkapseln gilt, vor allem Bleichmittel, Bleichaktivatoren, Enzyme, Vergrauungsinhibitoren, optische Aufheller sowie (chlor- bzw. peroxidstabile) Parfüm- und Farbstoffe in Frage.

Unter den als **Bleichmittel** dienenden, in Wasser Wasserstoffperoxid liefernden Verbindungen haben das Natriumperborat-Tetrahydrat und das Natriumperborat-Monohydrat eine besondere Bedeutung. Weitere Bleichmittel sind beispielsweise Peroxycarbonat, Citratperhydrate sowie Salze der Persäuren, wie Perbenzoate, Peroxyphthalate oder Diperoxydodecandisäure. Sie werden üblicherweise in Mengen von 8 bis 25 Gew.-% eingesetzt. Bevorzugt ist der Einsatz von Natriumperborat-Monohydrat in Mengen von 10 bis 20 Gew.-% und insbesondere von 10 bis 15 Gew.-%. Durch seine Fähigkeit, unter Ausbildung des Tetrahydrats freies Wasser binden zu können, trägt es zur Erhöhung der Stabilität des Mittels bei.

Beispiele für geeignete **Bleichaktivoren** sind mit Wasserstoffperoxid organische Persäuren bildende N-Acyl- bzw. O-Acyl-Verbindungen, vorzugsweise N,N'-tetraacylierte Diamine, ferner Carbonsäureanhydride und Ester von Polyolen wie Glucosepentaacetat. Der Gehalt der bleichmittelhaltigen Mittel an Bleichaktivatoren liegt in dem üblichen Bereich, vorzugsweise zwischen 1 und 10 Gew.-% und insbesondere zwischen 3 und 8 Gew.-%. Besonders bevorzugte Bleichaktivatoren sind N,N,N',N'-Tetraacetylethylendiamin und 1,5-Diacetyl-2,4-dioxo-hexahydro-1,3,5-triazin.

Als **Enzyme** kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Ihr Anteil kann etwa 0,2 bis etwa 2 Gew.-% betragen. Die Enzyme können an Trägerstoffen adsorbiert und/oder in Hüllsubstanzen eingebettet sein, um sie gegen vorzeitige Zersetzung zu schützen. Zusätzlich zu den mono- und polyfunktionellen Alkoholen und den Phosphonaten können die Mittel weitere Enzymstabilisatoren enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2-Gew.-%, bezogen auf das Enzym, stabilisiert sind. Besonders vorteilhaft ist jedoch der Einsatz von Borverbindungen, beispielsweise von Borsäure, Boroxid, Borax und anderen Alkalimetallboraten wie den Salzen der Orthoborsäure (H₃BO₃), der Metaborsäure (HBO₂) und der Pyroborsäure (Tetraborsäure H₂B4O₇).

Geeignete **Vergrauungsinhibitoren** sind wasserlösliche Kolloide meist organischer Natur, beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw.. Auch Polyvinylpyrrolidon ist brauchbar. Bevorzugt werden jedoch Celluloseether, wie Carboxymethylcellulose, Methylcellulose, Hydroxyalkylcellulose und Mischether, wie Methylhydroxyethylcellulose, Methylhydroxypropylcellulose, Methylcarboxymethylcellulose und deren Gemische sowie Polyvinylpyrrolidon, beispielsweise in Mengen von 0,1 bis 99 und vorzugsweise 1 bis 5 Gew.-%, bezogen auf die Mittel.

Als **optische Aufheller** können Derivate der Diaminostilbendisulfonsäure bzw. deren Alkalimetallsalze eingesetzt werden. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden. Ein besonders bevorzugter Farbstoff ist Tinolux® (Handelsprodukt der Ciba-Geigy).

Beispiele für **aktivchlorbeständige Duftstoffe** sind Citronellol (3,7-Dimethyl-6-octen-1-ol), Dimethyloctanol (3,7-Dimethyloctanol-1), Hydroxycitronellol (3,7-Dimethyloctane-1,7-diol), Mugol (3,7-Dimethyl-4,6-octatrien-3-ol), Mirsenol (2-Methyl-6-methylen-7-octen-2-ol), Terpinolen (p-Mentho-1,4(8)-dien), Ethyl-2-methylbutyrat, Phenylpropylalkohol, Galaxolid (1,3,4,6,7,8-hexahydro-4,6,6,7,8,8,-hexamethylcyclopental-2-benzopyran, Tonalid (7-Acetyl-1,1,3,4,4,6-hexamethyltetrahydronaphthalin), Rosenoxid, Linaloloxid, 2,6-Dimethyl-3-octanol, Tetrahydroethyllinalool, Tetrahydroethyllinalylacetat, o-sec-Butylcyclohexylacetat und Isolonediphorenepoxid sowie Isoborneal, Dihydroterpenöl, Isobornylacetat, Dihydroterpenylacetat). Weitere geeignete Duftstoffe sind die in der Europäischen Patentanmeldung **EP 0622451 A1** (Procter & Gamble) in den Spalten 3 und 4 genannten Stoffe.

Als **Farbpigmente** kommen neben anorganischen Stoffen, wie beispielsweise Eisen- oder Wismutoxiden, vor allem grüne Chlorophthalocyanine (Pigmosol® Grün, Hostaphine® Grün), gelbes Solar Yellow BG 300 (Sandoz), blaues Chlorophthalocyanin (Hostaphine® Blau) oder Cosmenyl® Blau in Frage.

### Ölphase

Als Ölphase, in der die Matrix feindispergiert wird, kommen die bereits im Kapitel "Wirkstoffe" genannten kosmetischen Ölkörper in Frage. Vorzugsweise arbeitet man in Paraffin- oder Pflanzenölen, wobei die Ölphase üblicherweise das 2 bis 5fache des Volumens der Matrix beträgt.

### Anionische Polymere

Die anionische Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

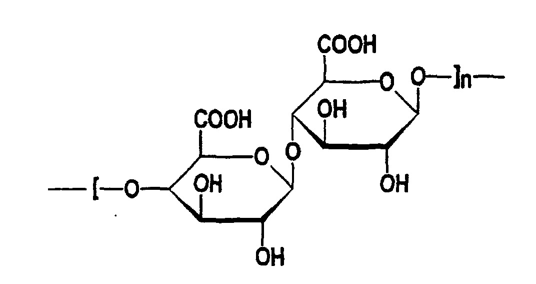

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage, wie sie beispielsweise in der deutschen Patentschrift **DE 3713099 C2** (L'Oreal) sowie der deutschen Patentanmeldung **DE 19604180 A1** (Henkel) beschrieben werden.

### Herstellverfahren

Zur Herstellung der neuen Mikrokapseln stellt man üblicherweise eine 1 bis 10, vorzugsweise 2 bis 5 Gew.-%ige wäßrige Lösung des Gelbildners, vorzugsweise des Agar-Agars her und erhitzt diese unter Rückfluß. In der Siedehitze, vorzugsweise bei 80 bis 100°C, wird eine zweite wäßrige Lösung zugegeben, welche das Chitosan in Mengen von 0,1 bis 2, vorzugsweise 0,25 bis 0,5 Gew.-% und den Wirkstoff in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wäßrigen oder wäßrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben.

Als **Emulgatoren** kommen beispielsweise nichtionogene Tenside aus mindestens einer der folgenden Gruppen in Frage:
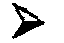 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
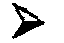 Alkyl- und/oder Alkenyloligoglykoside mit 8 bis 22 Kohlenstoffatomen im Alk(en)ylrest und deren ethoxylierte Analoga;
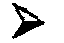 Anlagerungsprodukte von 1 bis 15 Mol Ethylenoxid an Ricinusöl und/oder gehärtetes Ricinusöl;
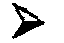 Anlagerungsprodukte von 15 bis 60 Mol Ethylenoxid an Ricinusöl und/oder gehärtetes Ricinusöl;
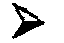 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
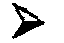 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
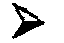 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
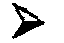 Mono-, Di- und Trialkylphosphate sowie Mono-, Di- und/oder Tri-PEG-alkylphosphate und deren Salze;
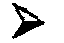 Wollwachsalkohole;
> Polysiloxan-Polyalkyl-Polyether-Copolymere bzw. entsprechende Derivate;
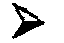 Polyalkylenglycole sowie
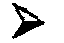 Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt. **Alkyl- und/oder Alkenyloligoglycoside,** ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Als Lösungsvermittler oder **Hydrotrope** eignen sich beispielsweise Ethanol, Isopropylalkohol, oder Polyole eingesetzt werden. Letztere besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
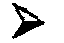 Glycerin;
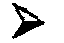 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
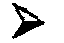 technische Oligoglyceringemische mit einem Eigenkondensationsgrad von 1,5 bis 10 wie etwa technische Diglyceringemische mit einem Diglyceringehalt von 40 bis 50 Gew.-%;
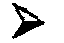 Methyolverbindungen, wie insbesondere Trimethylolethan, Trimethylolpropan, Trimethylolbutan, Pentaerythrit und Dipentaerythrit;
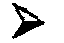 Niedrigalkylglucoside, insbesondere solche mit 1 bis 8 Kohlenstoffen im Alkylrest, wie beispielsweise Methyl- und Butylglucosid;
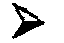 Zuckeralkohole mit 5 bis 12 Kohlenstoffatomen, wie beispielsweise Sorbit oder Mannit,
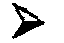 Zucker mit 5 bis 12 Kohlenstoffatomen, wie beispielsweise Glucose oder Saccharose;
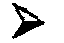 Aminozucker, wie beispielsweise Glucamin;
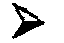 Dialkoholamine, wie Diethanolamin oder 2-Amino-1,3-propandiol.

Die Konzentration der Emulgatoren kann bezogen auf die Wirkstoffe 1 bis 20 und vorzugsweise 5 bis 10 Gew.-% betragen. Die Menge an Lösungsvermittler richtet sich ausschließlich nach der Wasserlöslichkeit bzw. Wasserdispergierbarkeit der Wirkstoffe.

Nach der Herstellung der Matrix aus Gelbildner, Chitosan und Wirkstoff wird die Matrix in einer Ölphase unter starker Scherung sehr fein dispergiert, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im dritten Schritt erfolgt dann die eigentliche Verkapselung, d.h. die Ausbildung der Hüllmembran durch Inkontaktbringen des Chitosans in der Matrix mit den anionischen Polymeren. Hierzu empfiehlt es sich, die in der Ölphase dispergierte Matrix bei einer Temperatur im Bereich von 40 bis 100, vorzugsweise 50 bis 60 °C mit einer wäßrigen, etwa 0,1 bis 3 und vorzugsweise 0,25 bis 0,5 Gew.-%ige wäßrigen Lösung des Anionpolymers, vorzugsweise des Alginats zu waschen und dabei gleichzeitig die Ölphase zu entfernen. Die dabei resultierenden wäßrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise 1 bis 3 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig.

### Kosmetische und/oder pharmazeutische Zubereitungen

Die Mikrokapseln der vorliegenden Erfindung dienen zur Herstellung oberflächenaktiver Mittel, in einer ersten Ausführungsform insbesondere zur Herstellung von kosmetischen und/oder pharmazeutischen Zubereitungen, als da sind Haarshampoos, Haarlotionen, Schaumbäder, Duschbäder, Cremes, Gele, Lotionen, alkoholische und wäßrig/alkoholische Lösungen, Emulsionen, Wachs/Fett-Massen, Stiftpräparaten, Pudem oder Salben. Diese Mittel können neben den Mikrokapseln, die in Mengen von 0,1 bis 99 und vorzugsweise 1 bis 5 Gew.-% - bezogen auf die Zubereitungen - enthalten sein können, als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Licht-schutzfaktoren, Antioxidantien, Hydrotrope, Konservierungsmittel, Insektenrepellentien, Selbstbräuner, Solubilisatoren, Parfümöle, Farbstoffe und dergleichen aufweisen. Eine Reihe dieser Hilfsstoffe sind schon in den vorherigen Kapiteln näher erläutert worden, so daß an dieser Stelle auf eine Widerholung verzichtet wird.

Typische Beispiele für geeignete milde, d.h. besonders hautverträgliche **Tenside** sind Fettalkoholpolyglycolethersulfate, Monoglyceridsulfate, Mono- undloder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine undloder Proteinfettsäurekondensate, letztere vorzugsweise auf Basis von Weizenproteinen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, VinylpyrrolidonNinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperaturmethode.

### Detergenszubereitungen

In einer weiteren Ausführungsform der Erfindung dienen die Mikrokapseln zur Herstellung von Detergentien, speziell Wasch-, Spül-, Reinigungs- und Avivagemitteln, in denen sie ebenfalls in Mengen von 0,1 bis 99 und vorzugsweise 1 bis 5 Gew.-% - bezogen auf die Zubereitungen - enthalten sein können; vorzugsweise handelt es sich dabei um wäßrige oder wäßrig-alkoholische Mittel. Solche Flüssigwaschmittel können einen nicht wäßrigen Anteil im Bereich von 5 bis 50 und vorzugsweise 15 bis 35 Gew.-% aufweisen. Im einfachsten Fall handelt es sich um wäßrige Lösungen der genannten Tensidmischungen. Bei den Flüssigwaschmitteln kann es sich aber auch um im wesentlichen wasserfreie Mittel handeln. Dabei bedeutet "im wesentlichen wasserfrei", daß das Mittel vorzugsweise kein freies, nicht als Kristallwasser oder in vergleichbarer Form gebundenes Wasser enthält. In einigen Fällen sind geringe Menge an freiem Wasser tolerierbar, insbesondere in Mengen bis zu 5 Gew.-%. Die Flüssigwaschmittel können neben den genannten Tensiden noch weitere typische Inhaltsstoffe, wie beispielsweise Lösungsmittel, Hydrotrope, Bleichmittel, Builder, Viskositätsregulatoren, Enzyme, Enzymstabilisatoren, optische Aufheller, Soil repellants, Schauminhibitoren, anorganische Salze sowie Duft- und Farbstoffe aufweisen, unter der Voraussetzung, daß diese im wäßrigen Milieu hinreichend lagerstabil sind. Auch hier wurden eine Reihe der genannten Hilfsstoffe bereits in vorhergehenden Kapiteln abgehandelt, so daß sich eine Wiederholung erübrigt.

Als organische **Lösungsmittel** kommen beispielsweise mono- und/oder polyfunktionelle Alkohole mit 1 bis 6 Kohlenstoffatomen, vorzugsweise mit 1 bis 4 Kohlenstoffatomen in Frage. Bevorzugte Alkohole sind Ethanol, 1,2-Propandiol, Glycerin sowie deren Gemische. Die Mittel enthalten vorzugsweise 2 bis 20 Gew.-% und insbesondere 5 bis 15 Gew.-% Ethanol oder ein beliebiges Gemisch aus Ethanol und 1,2-Propandiol oder insbesondere aus Ethanol und Glycerin. Ebenso ist es möglich, daß die Zubereitungen entweder zusätzlich zu den mono- und/oder polyfunktionellen Alkoholen mit 1 bis 6 Kohlenstoffatomen oder allein Polyethylenglykol mit einer relativen Molekülmasse zwischen 200 und 2000, vorzugsweise bis 600 in Mengen von 2 bis 17 Gew.-% enthalten. Als Hydrotrope können beispielsweise Toluolsulfonat, Xylolsulfonat, Cumolsulfonat oder deren Mischungen eingesetzt werden.

Geeignete **Builder** sind Ethylendiamintetraessigsäure, Nitrilotriessigsäure, Citronensäure sowie anorganische Phosphonsäuren, wie z.B. die neutral reagierenden Natriumsalze von 1-Hydroxyethan-1,1,diphosphonat, die in Mengen von 0,5 bis 5, vorzugsweise 1 bis 2 Gew.-% zugegen sein können.

Als **Viskositätsregulatoren** können beispielsweise gehärtetes Rizinusöl, Salze von langkettigen Fettsäuren, die vorzugsweise in Mengen von 0 bis 5 Gew.-% und insbesondere in Mengen von 0,5 bis 2 Gew.-%, beispielsweise Natrium-, Kalium-, Aluminium-, Magnesium- und Titanstearate oder die Natrium- undloder Kaliumsalze der Behensäure, sowie weitere polymere Verbindungen eingesetzt werden. Zu den letzteren gehören bevorzugt Polyvinylpyrrolidon, Urethane und die Salze polymerer Polycarboxylate, beispielsweise homopolymerer oder copolymerer Polyacrylate, Polymethacrylate und insbesondere Copolymere der Acrylsäure mit Maleinsäure, vorzugsweise solche aus 50 % bis 10 % Maleinsäure. Die relative Molekülmasse der Homopolymeren liegt im allgemeinen zwischen 1000 und 100000, die der Copolymeren zwischen 2000 und 200000, vorzugsweise zwischen 50000 bis 120000, bezogen auf die freie Säure. Insbesondere sind auch wasserlösliche Polyacrylate geeignet, die beispielsweise mit etwa 1 % eines Polyallylethers der Sucrose quervernetzt sind und die eine relative Molekülmasse oberhalb einer Million besitzen. Beispiele hierfür sind die unter dem Namen Carbopol® 940 und 941 erhältlichen Polymere mit verdickender Wirkung. Die quervernetzten Polyacrylate werden vorzugsweise in Mengen nicht über 1 Gew.-%, vorzugsweise in Mengen von 0,2 bis 0,7 Gew.-% eingesetzt. Die Mittel können zusätzlich etwa 5 bis 20 Gew.-% eines partiell veresterten Copolymerisats enthalten, wie es in der europäischen Patentanmeldung **EP 0367049 A** beschrieben ist. Diese partiell veresterten Polymere werden durch Copolymerisation von (a) mindestens einem C₄-C₂₈-Olefin oder Mischungen aus mindestens einem C₄-C₂₈-Olefin mit bis zu 20 Mol-% C₁-C₂₈-Alkylvinylethem und (b) ethylenisch ungesättigten Dicarbonsäureanhydriden mit 4 bis 8 Kohlenstoffatomen im Molverhältnis 1 : 1 zu Copolymerisaten mit K-Werten von 6 bis 100 und anschließende partielle Veresterung der Copolymerisate mit Umsetzungsprodukten wie C₁-C₁₃-Alkoholen, C₈-C₂₂-Fettsäuren, C₁-C₁₂-Alkylphenolen, sekundären C₂-C₃₀-Aminen oder deren Mischungen mit mindestens einem C₂-C₄-Alkylenoxid oder Tetrahydrofuran sowie Hydrolyse der Anhydridgruppen der Copolymerisate zu Carboxylgruppen erhalten, wobei die partielle Veresterung der Copolymerisate soweit geführt wird, daß 5 bis 50 % der Carboxylgruppen der Copolymerisate verestert sind. Bevorzugte Copolymerisate enthalten als ethylenisch ungesättigtes Dicarbonsäureanhydrid Maleinsäureanhydrid. Die partiell veresterten Copolymerisate können entweder in Form der freien Säure oder vorzugsweise in partiell oder vollständig neutralisierter Form vorliegen. Vorteilhafterweise werden die Copolymerisate in Form einer wäßrigen Lösung, insbesondere in Form einer 40 bis 50 Gew.-%igen Lösung eingesetzt. Die Copolymerisate leisten nicht nur einen Beitrag zur Primär- und Sekundärwaschleistung des flüssigen Wasch- und Reinigungsmittels, sondern bewirken auch eine gewünschte Viskositätsemiedrigung der konzentrierten flüssigen Waschmittel. Durch den Einsatz dieser partiell veresterten Copolymerisate werden konzentrierte wäßrige Flüssigwaschmittel erhalten, die unter dem alleinigen Einfluß der Schwerkraft und ohne Einwirkung sonstiger Scherkräfte fließfähig sind. Vorzugsweise beinhalten die konzentrierten wäßrigen Flüssigwaschmittel partiell veresterte Copolymerisate in Mengen von 5 bis 15 Gew.-% und insbesondere in Mengen von 8 bis 12 Gew.-%.

Als schmutzabweisende Polymere (**"soil repellants"**) kommen solche Stoffe in Frage, die vorzugsweise Ethylenterephthalat- und/oder Polyethylenglycolterephthalatgruppen enthalten, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt insbesondere im Bereich von 750 bis 5000, d.h., der Ethoxylierungsgrad der Polyethylenglycolgruppenhaltigen Polymere kann ca. 15 bis 100 betragen. Die Polymere zeichnen sich durch ein durchschnittliches Molekulargewicht von etwa 5000 bis 200.000 aus und können eine Block-, vorzugsweise aber eine Random-Struktur aufweisen. Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymere, die verknüpfende Polyethylenglycoleinheiten mit einem Molekulargewicht von 750 bis 5000, vorzugsweise von 1000 bis etwa 3000 und ein Molekulargewicht der Polymere von etwa 10.000 bis etwa 50.000 aufweisen. Beispiele für handelsübliche Polymere sind die Produkte Milease® T (ICI) oder Repelotex® SRP 3 (Rhône-Poulenc).

Beim Einsatz in maschinellen Waschverfahren kann es von Vorteil sein, den Mitteln übliche **Schaum inhibitoren** zuzusetzen. Hierfür eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, z.B. solche aus Silikonen, Paraffinen oder Wachsen. Vorzugsweise sind die Schauminhibitoren, insbesondere silikon- oder paraffinhaltige Schauminhibitoren, an eine granulare, in Wasser lösliche bzw. dispergierbare Trägersubstanz gebunden. Insbesondere sind dabei Mischungen aus Paraffinen und Bistearylethylendiamiden bevorzugt.

Der pH-Wert der Mittel beträgt im allgemeinen 7 bis 10,5, vorzugsweise 7 bis 9,5 und insbesondere 7 bis 8,5. Die Einstellung höherer pH-Werte, beispielsweise oberhalb von 9, kann durch den Einsatz geringer Mengen an Natronlauge oder an alkalischen Salzen wie Natriumcarbonat oder Natriumsilicat erfolgen. Die Flüssigwaschmittel weisen im allgemeinen Viskositäten zwischen 150 und 10000 mPas (Brookfield-Viskosimeter, Spindel 1, 20 Umdrehungen pro Minute, 20°C) auf. Dabei sind bei den im wesentlichen wasserfreien Mitteln Viskositäten zwischen 150 und 5000 mPas bevorzugt. Die Viskosität der wäßrigen Mittel liegt vorzugsweise unter 2000 mPas und liegt insbesondere zwischen 150 und 1000 mPas.

In einer letzten Ausführungsform eignen sich beispielsweise mit Aromen beladene Mikrokapseln zur Herstellung von Lebensmitteln.

### Beispiele

**Beispiel 1.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g Paraffinöl, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel 2.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g Squalan, 0,5 g Phenonip® und 0,5 g Ceteareth-20 in ad 100 g Wasser versetzt. Die erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel 3.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g Panthenol, 0,5 g Phenonip® in ad 100 g Wasser versetzt. Die erhaltene Matrix wurde filtriert, auf 50 °C temperiert und unter starken Rühren im 3fachen Volumen Sojaöl, das zuvor auf 15 °C gekühlt worden war, fdispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel 4.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g β-Carotin, 0,5 g Phenonip® in ad 100 g Wasser versetzt. Die erhaltene Matrix wurde filtriert, auf 50 °C temperiert und unter starken Rühren im 2,5fachen Volumen Sojaöl, das zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel 5.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g Tocopherolacetat, 0,5 g Phenonip® in ad 100 g Wasser versetzt. Die erhaltene Matrix wurde filtriert, auf 50 °C temperiert und unter starken Rühren im 2fachen Volumen Dicaprylylether, der zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel 6.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g Ascorbinsäure, 0,5 g Phenonip® in ad 100 g Wasser versetzt. Die erhaltene Matrix wurde filtriert, auf 50 °C temperiert und unter starken Rühren im 2,5fachen Volumen Cocoglycerides, die zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel 7.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g Kojisäure, 0,5 g Phenonip® in ad 100 g Wasser versetzt. Die erhaltene Matrix wurde filtriert, auf 50 °C temperiert und unter starken Rühren im 4fachen Volumen Oleyloleat, das zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel 8.** In einem 500-ml-Dreihalskolben mit Rührer und Rückflußkühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g Dehyquart F® 75 (Distearoylethyl hydroxyethylmonium Methosulfate and Cetearyl Alcohol, Henkel KGaA), 0,5 g Phenonip® in ad 100 g Wasser versetzt. Die erhaltene Matrix wurde filtriert, auf 50 °C temperiert und unter starken Rühren im 2,5fachen Volumen Octyldodecanol, das zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Natriumalginat und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

**Beispiel 9.** In einem 500-ml-Dreihalskolben mit Rührer und Rückflußkühler wurden in der Siedehitze 3 g Gelatine in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Henkel KGaA, Düsseldorf/FRG), 10 g Dehyquart F® 75 (Distearoylethyl Hydroxyethylmonium Methosulfate and Cetearyl Alcohol, Henkel KGaA), 0,5 g Phenonip® in ad 100 g Wasser versetzt. Die erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer wäßrigen Lösung enthaltend 1 Gew.-% Natriumlaurylsulfat und 0,5 Gew.-% Hydagen® SCD (succinyliertes Chitosan, Henkel KGaA) und dann mehrfach mit einer 0,5 Gew.-%igen wäßrigen Phenoniplösung gewaschen, wobei die Ölphase entfernt wurde. Nach dem Sieben wurde eine wäßrige Zubereitung erhalten, die 8 Gew.-% Mikrokapseln mit einem mittleren Durchmesser von 1 mm enthielt.

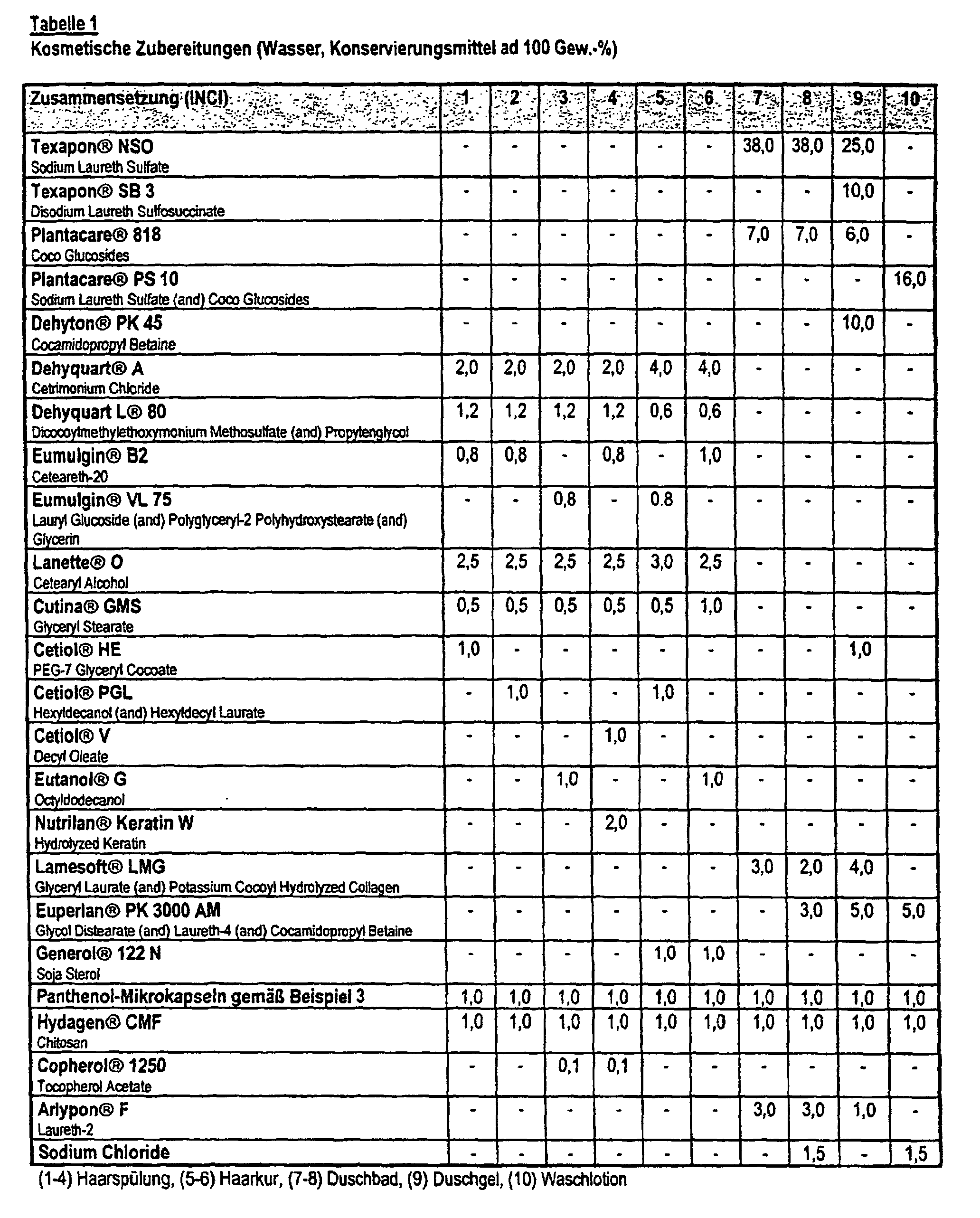

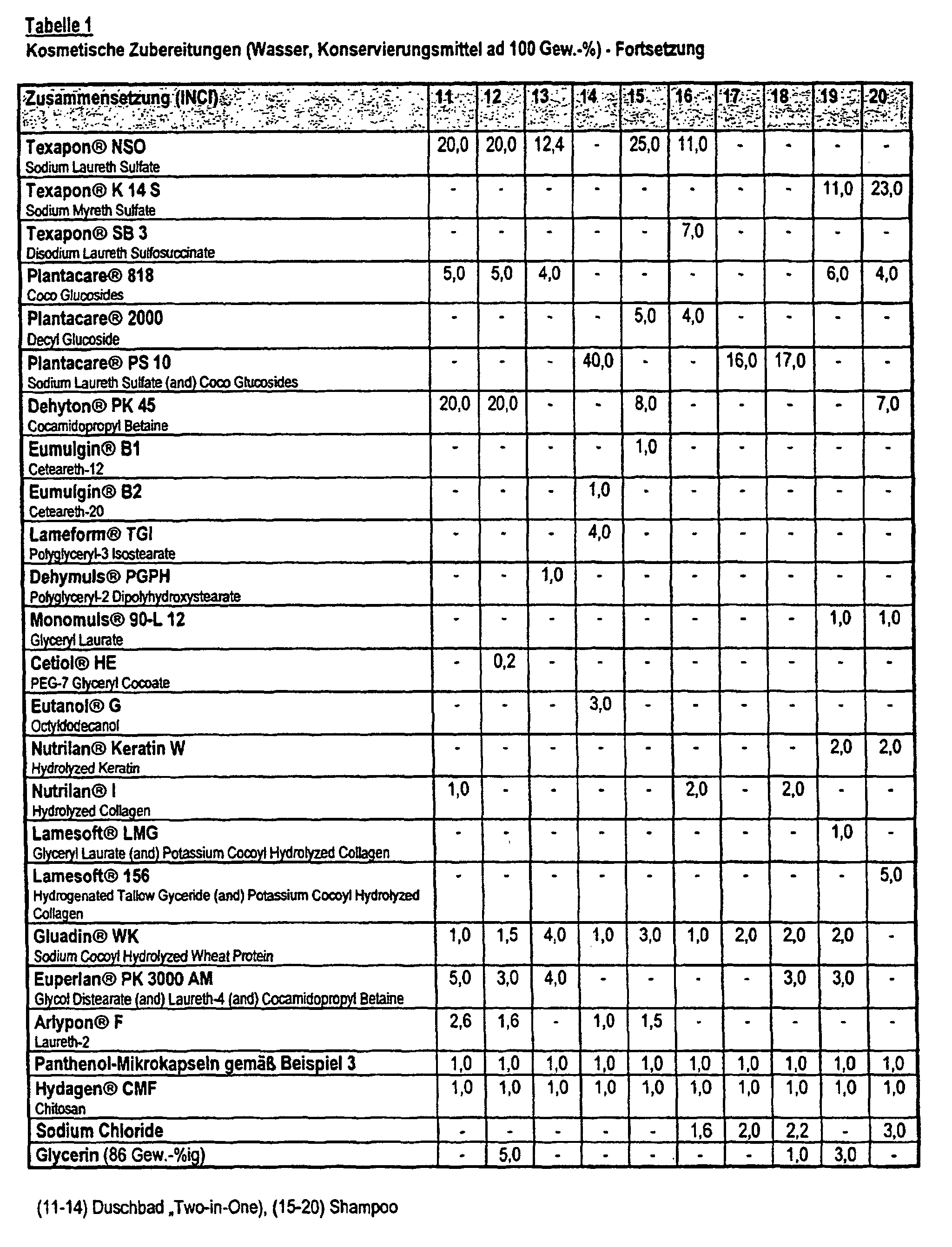

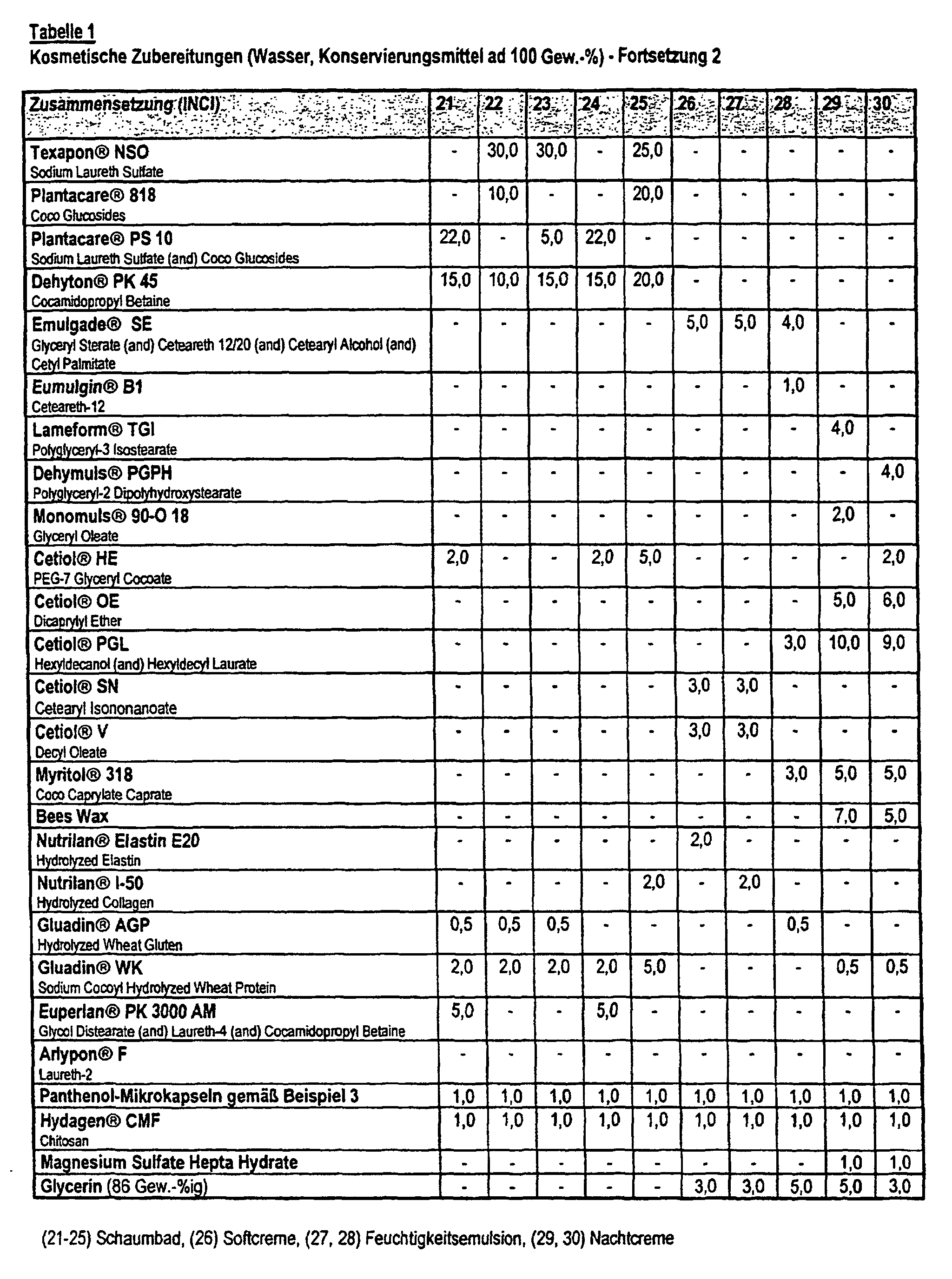

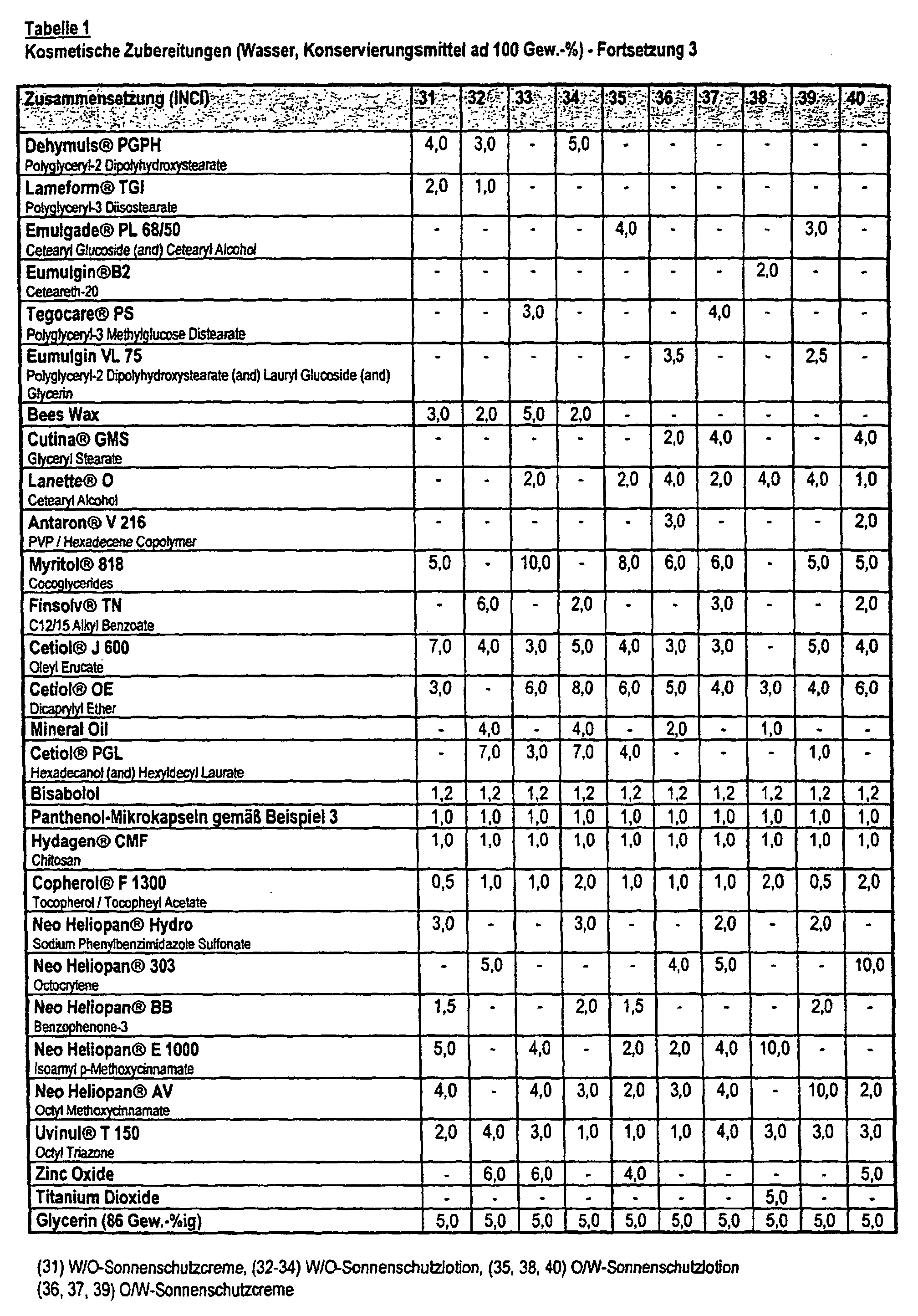

## Patentansprüche

1. Mikrokapseln mit mittleren Durchmessern im Bereich von 0,1 bis 5 mm, bestehend aus einer Hüllmembran und einer mindestens einen Wirkstoff enthaltenden Matrix, dadurch erhältlich, daß man
(a) aus Gelbildnern, Chitosanen und Wirkstoffen eine Matrix zubereitet,
(b) die Matrix in einer Ölphase dispergiert,
(c) die dispergierte Matrix mit wäßrigen Lösungen anionischer Polymere behandelt und dabei die Ölphase entfernt.

2. Verfahren zur Herstellung von Mikrokapseln mit mittleren Durchmessern im Bereich von 0,1 bis 5 mm, bestehend aus einer Hüllmembran und einer mindestens einen Wirkstoff enthaltenden Matrix, bei dem man
(a) aus Gelbildnern, Chitosanen und Wirkstoffen eine Matrix zubereitet,
(b) die Matrix in einer Ölphase dispergiert,
(c) die dispergierte Matrix mit wäßrigen Lösungen anionischer Polymere behandelt und dabei die Ölphase entfernt.

3. Verfahren nach Anspruch 2, **dadurch gekennzeichnet, daß** man als Gelbildner Heteropolysaccharide oder Proteine einsetzt.

4. Verfahren nach Anspruch 3, **dadurch gekennzeichnet, daß** man als Heteropolysaccharide Agarosen, Agar-Agar, Pektine, Xanthane sowie deren Gemische einsetzt.

5. Verfahren nach Anspruch 3, **dadurch gekennzeichnet, daß** man als Proteine Gelatine einsetzt.

6. Verfahren nach mindestens einem der Ansprüche 2 bis 5, **dadurch gekennzeichnet, daß** man Chitosane einsetzt, die ein mittleres Molekulargewicht im Bereich von 10.000 bis 500.000 bzw. 800.000 bis 1.200.000 Dalton aufweisen.

7. Verfahren nach mindestens einem der Ansprüche 2 bis 6, **dadurch gekennzeichnet, daß** man Wirkstoffe einsetzt, die ausgewählt sind aus der Gruppe, die gebildet wird von Tensiden, kosmetischen Ölen, Perlglanzwachsen, Stabilisatoren, biogenen Wirkstoffen, Deodorantien, Antitranspirantien, Antischuppenmitteln, UV-Lichtschutzfaktoren, Antioxidantien, Konservierungsmitteln, Insektenrepellentien, Selbstbräunem, Parfümölen, Aromastoffen, Bleichmitteln, Bleichaktivatoren, Enzymen, Vergrauungsinhibitoren, optischen Aufhellem und Farbstoffen.

8. Verfahren nach mindestens einem der Ansprüche 2 bis 7, **dadurch gekennzeichnet, daß** man als anionische Polymere Salze der Alginsäure oder anionische Chitosanderivate einsetzt.

9. Verfahren nach mindestens einem der Ansprüche 2 bis 8, **dadurch gekennzeichnet, daß** man die Mikrokapseln - bezogen auf das Kapselgewicht - mit 0,1 bis 25 Gew.-% Wirkstoff belädt.

10. Verfahren nach mindestens einem der Ansprüche 2 bis 9, **dadurch gekennzeichnet, daß** man bei der Herstellung der Matrix Emulgatoren und/oder Viskositätsregulatoren mitverwendet.

11. Verfahren nach mindestens einem der Ansprüche 2 bis 10, **dadurch gekennzeichnet, daß** man die Matrix bei Temperaturen im Bereich von 40 bis 100 °C herstellt.

12. Verfahren nach mindestens einem der Ansprüche 2 bis 11, **dadurch gekennzeichnet, daß** man die Matrix im 2 bis 5fachen Volumen der Ölphase dispergiert.

13. Verfahren nach mindestens einem der Ansprüche 2 bis 12, **dadurch gekennzeichnet, daß** man die auf 40 bis 60 °C erwärmte Matrix in einer auf 10 bis 20 °C gekühlten Ölphase dispergiert.

14. Verfahren nach mindestens einem der Ansprüche 1 bis 13, **dadurch gekennzeichnet, daß** man die in der Ölphase dispergierte Matrix mit 0,1 bis 3 Gew.-%igen wäßrigen Lösungen der anionischen Polymere behandelt.

15. Verfahren nach mindestens einem der Ansprüche 2 bis 14, **dadurch gekennzeichnet, daß** man die in der Ölphase feindispergierte Matrix bei Temperaturen im Bereich von 40 bis 100 °C mit den wäßrigen Lösungen der anionischen Polymere behandelt.

16. Verfahren nach Anspruch 15, **dadurch gekennzeichnet, daß** man die in der Ölphase dispergierte Matrix mit wäßrigen Lösungen der anionischen Polymere wäscht und die Ölphase dabei gleichzeitig entfernt.

17. Verfahren nach Anspruch 16, **dadurch gekennzeichnet, daß** man bei der Wäsche wäßrige Zubereitungen herstellt, deren Anteil an Mikrokapseln schließlich im Bereich von 1 bis 10 Gew.-% liegt.

18. Verfahren nach mindestens einem der Ansprüche 2 bis 17, **dadurch gekennzeichnet, daß** man die Zubereitungen ständig rührt.

19. Verwendung von Mikrokapseln nach Anspruch 1 zur Herstellung von kosmetischen und/oder pharmazeutischen Zubereitungen.

20. Verwendung von Mikrokapseln nach Anspruch 1 zur Herstellung von Wasch-, Spül-, Reinigungsund Avivagemitteln.

21. Verwendung von Mikrokapseln nach Anspruch 1 zur Herstellung von Lebensmitteln.

22. Verwendung nach mindestens einem der Ansprüche 19 bis 21 , **dadurch gekennzeichnet, daß** man die Mikrokapseln in Mengen von 0,1 bis 99 Gew.-% - bezogen auf die Zubereitungen - einsetzt.

## Claims

1. Microcapsules with mean diameters of 0.1 to 5 mm consisting of a membrane and a matrix containing at least one active principle and obtainable by
(a) preparing a matrix from gel formers, chitosans and active principles,
(b) dispersing the matrix in an oil phase,
(c) treating the dispersed matrix with aqueous solutions of anionic polymers and removing the oil phase in the process.

2. A process for the production of microcapsules with mean diameters of 0.1 to 5 mm consisting of a membrane and a matrix containing at least one active principle, **characterized in that** it comprises the steps of
(a) preparing a matrix from gel formers, chitosans and active principles,
(b) dispersing the matrix in an oil phase and
(c) treating the dispersed matrix with aqueous solutions of anionic polymers and removing the oil phase in the process.

3. A process as claimed in claim 2, **characterized in that** heteropolysaccharides or proteins are used as gel the formers.

4. A process as claimed in claim 3, **characterized in that** agaroses, agar agar, pectins, xanthans and mixtures thereof are used as the heteropolysaccharides.

5. A process as claimed in claim 3, **characterized in that** gelatine is used as the protein.

6. A process as claimed in at least one of claims 2 to 5, **characterized in that** chitosans with an average molecular weight of 10,000 to 500,000 or 800,000 to 1,200,000 dalton are used.

7. A process as claimed in at least one of claims 2 to 6, **characterized in that** active principles selected from the group consisting of surfactants, cosmetic oils, pearlizing waxes, stabilizers, biogenic agents, deodorants, antiperspirants, antidandruff agents, UV protection factors, antioxidants, preservatives, insect repellents, self-tanning agents, perfume oils, flavors, bleaching agents, bleach activators, enzymes, redeposition inhibitors, optical brighteners and dyes are used.

8. A process as claimed in at least one of claims 2 to 7, **characterized in that** salts of alginic acid or anionic chitosan derivatives are used as the anionic polymers.

9. A process as claimed in at least one of claims 2 to 8, **characterized in that** the microcapsules are charged with 0.1 to 25% by weight, based on their weight, of active principle.

10. A process as claimed in at least one of claims 2 to 9, **characterized in that** emulsifiers and/or viscosity adjusters are used in the production of the matrix.

11. A process as claimed in at least one of claims 2 to 10, **characterized in that** the matrix is produced at temperatures of 40 to 100°C.

12. A process as claimed in at least one of claims 2 to 11, **characterized in that** the matrix is dispersed in 2 to 5 times its volume of the oil phase.

13. A process as claimed in at least one of claims 2 to 12, **characterized in that** the matrix heated to 40 to 60°C is dispersed in an oil phase cooled to 10 to 20°C.

14. A process as claimed in at least one of claims 1 to 13, **characterized in that** the matrix dispersed in the oil phase is treated with 0.1 to 3% by weight aqueous solutions of the anionic polymers.

15. A process as claimed in at least one of claims 2 to 14, **characterized in that** the matrix finely dispersed in the oil phase is treated with the aqueous solutions of the anionic polymers at temperatures of 40 to 100°C.

16. A process as claimed in claim 15, **characterized in that** the matrix dispersed in the oil phase is washed with aqueous solutions of the anionic polymers and the oil phase is removed in the process.

17. A process as claimed in claim 16, **characterized in that** aqueous preparations ultimately containing 1 to 10% by weight of microcapsules are prepared in the washing step.

18. A process as claimed in at least one of claims 2 to 17, **characterized in that** the preparations are continuously stirred.

19. The use of the microcapsules claimed in claim 1 for the production of cosmetic and/or pharmaceutical preparations.

20. The use of the microcapsules claimed in claim 1 for the production of laundry detergents, dishwasher detergents, cleaners and softeners.

21. The use of the microcapsules claimed in claim 1 for the production of foods.

22. The use claimed in at least one of claims 19 to 21, **characterized in that** the microcapsules are used in quantities of 0.1 to 99% by weight, based on the preparations.

## Revendications

1. Microcapsules ayant des diamètres moyens dans la zone de 0,1 à 5 mm, constituées d'une membrane d'enrobage et d'une matrice contenant au moins un principe actif, que l'on peut obtenir en ce qu'
a) on prépare une matrice à base d'agents gélifiants, de chitosans et de principes actifs,
b) on met en dispersion la matrice dans une phase huileuse,
c) on traite la matrice mise en dispersion, avec des solutions aqueuses de polymères anioniques et de cette façon on élimine la phase huileuse.

2. Procédé de préparation de microcapsules ayant des diamètres moyens dans la zone de 0,1 à 5 mm qui consistent en une membrane d'enrobage et en une matrice contenant au moins un principe actif, dans lequel,
a) on prépare une matrice à base d'agents gélifiants, de chitosans et de principes actifs,
b) on met en dispersion la matrice dans une phase huileuse,
c) on traite la matrice mise en dispersion, avec des solutions aqueuses de polymères anioniques et de cette façon on élimine la phase huileuse.

3. Procédé selon la revendication 2,
**caractérisé en ce que**
comme agents gélifiants, on met en oeuvre des hétéropolysaccharides ou des protéines.

4. Procédé selon la revendication 3,
**caractérisé en ce que**
comme hétéropolysaccharides, on met en oeuvre des agaroses, de l'agar-agar, des pectines, de la gomme xanthane, ainsi que leurs mélanges.

5. Procédé selon la revendication 3,
**caractérisé en ce qu'**
on met en oeuvre en tant que protéine, de la gélatine.

6. Procédé selon au moins l'une quelconque des revendications 2 à 5,
**caractérisé en ce qu'**
on met en oeuvre des chitosanes qui possèdent un poids moléculaire moyen dans la zone de 10.000 à 500.000 ou de 800.000 à 1.200.000 daltons.

7. Procédé selon au moins l'une quelconque des revendications 2 à 6,
**caractérisé en ce qu'**
on met en oeuvre des principes actifs choisis dans le groupe formé des agents tensioactifs, des huiles cosmétiques, des cires à éclat nacré, des agents stabilisants, des principes actifs biogènes, des agents désodorisants, des agents antitranspirants, des agents antipelliculaires, des facteurs de protection contre la lumière UV, des agents antioxydants, des agents conservateurs, des agents répulsifs pour les insectes, des agents autobronzants, des essences de parfum, des substances aromatiques, des agents blanchissants, des activateurs de blanchiment, des enzymes, des inhibiteurs de grisaillement, des azurants optiques et des colorants.

8. Procédé selon au moins l'une quelconque des revendications 2 à 7,
**caractérisé en ce qu'**
on met en oeuvre en tant que polymères anioniques, des sels d'acide alginique ou des dérivés anioniques de chitosan.

9. Procédé selon au moins l'une quelconque des revendications 2 à 8,
**caractérisé en ce qu'**
on charge les microcapsules avec 0,1 à 25 % en poids de principe actif, rapporté au poids de la capsule.

10. Procédé selon au moins l'une quelconque des revendications 2 à 9,
**caractérisé en ce qu'**
on utilise conjointement lors de la production de la matrice, des agents émulsionnants et/ou des régulateurs de viscosité.

11. Procédé selon au moins l'une quelconque des revendications 2 à 10,
**caractérisé en ce qu'**
on produit la matrice à des températures dans la plage de 40 à 100°C.

12. Procédé selon au moins l'une quelconque des revendications 2 à 11,
**caractérisé en ce qu'**
on met en dispersion la matrice dans un volume double à quintuple de phase huileuse.

13. Procédé selon au moins l'une quelconque des revendications 2 à 12,
**caractérisé en ce qu'**
on met en dispersion la matrice chauffée de 40 à 60°C dans une phase huileuse refroidie de 10 à 20°C.

14. Procédé selon au moins l'une quelconque des revendications 2 à 13,
**caractérisé en ce qu'**
on traite la matrice mise en dispersion dans la phase huileuse avec des solutions aqueuses de 0,1 à 3 % en poids des polymères anioniques.

15. Procédé selon au moins l'une quelconque des revendications 2 à 14,
**caractérisé en ce qu'**
on traite la matrice mise en fine dispersion dans la phase huileuse, avec les solutions aqueuses de polymères anioniques à des températures dans la plage de 40 à 400°C.

16. Procédé selon la revendication 15,
**caractérisé en ce qu'**
on lave la matrice mise en dispersion dans la phase huileuse avec des solutions aqueuses des polymères anioniques et on élimine de cette façon simultanément la phase huileuse.

17. Procédé selon la revendication 16,
**caractérisé en ce qu'**
au cours des lavages, on produit des préparations aqueuses dont la proportion en microcapsules se situe finalement dans la zone de 1 à 10 % en poids.

18. Procédé selon au moins l'une quelconque des revendications 1 à 17,
**caractérisé en ce qu'**
on agite constamment les préparations.

19. Utilisation des microcapsules selon la revendication 1, en vue de la production de préparations cosmétiques et/ou pharmaceutiques.

20. Utilisation des microcapsules selon la revendication 1, en vue de la production de produits de lavage, de rinçage, de nettoyage et d'avivage.

21. Utilisation des microcapsules selon la revendication 1 pour la préparation de produits alimentaires.

22. Utilisation selon au moins l'une quelconque des revendications 19 à 21,
**caractérisée en ce qu'**
on met en oeuvre les microcapsules en quantités allant de 0,1 à 99 % en poids rapporté aux préparations.
